Europäisches Patentamt

**European Patent Office**

Office européen des brevets

(11) Publication number: **0 196 813 B1**

# EUROPEAN PATENT SPECIFICATION

(45) Date of publication of patent specification: **29.05.91**

(51) Int. Cl.⁵: **A61K 33/18**, A61K 47/00, A01N 59/12, A61K 9/52

(21) Application number: **86301898.2**

(22) Date of filing: **14.03.86**

The file contains technical information submitted after the application was filed and not included in this specification

(54) **A process for the preparation of a free flowing, homogeneous, iodophor containing wound powder.**

(30) Priority: **18.03.85 NL 8500774**

(43) Date of publication of application:
**08.10.86 Bulletin 86/41**

(45) Publication of the grant of the patent:
**29.05.91 Bulletin 91/22**

(84) Designated Contracting States:
**AT BE CH DE FR GB IT LI LU NL SE**

(56) References cited:
**EP-A- 0 124 774**      **EP-A- 0 172 984**
**DE-A- 3 131 071**      **FR-A- 2 454 303**
**GB-A- 2 084 464**      **US-A- 4 238 477**

(73) Proprietor: **Euroceltique SA**
**122 Boulevard de la Petrusse**
**Luxembourg(LU)**

(72) Inventor: **Jauw, Tjoe Hang**
**Theo Mann Bouwmeesterhof no. 4**
**NL-1065 HS Amsterdam(NL)**

(74) Representative: **Lamb, John Baxter et al**
**MARKS & CLERK 57/60 Lincoln's Inn Fields**
**London WC2A 3LS(GB)**

## Description

This invention relates to a process for the preparation of a free flowing, homogeneous, iodophor containing wound powder.

Iodophors are physiologically acceptable complexes of iodine with organic polymers, in which the germicidal and microbiocidal activity of elemental iodine is maintained. In the main, they are water-soluble.

Amongst the most effective (microbiocidally) of these iodophors are the complexes of iodine with non-ionic, non-detergent (non-surface active) organic polymers, such as polydextrose or, which is most widely used, polyvinylpyrrolidone (povidone).

Iodophors, for example polydextrose iodine and povidone iodine, may be dispensed in various kinds of semi-solid and liquid pharmaceutical preparations, such as solutions, ointments and aerosols. In the treatment of wounds, however, a powder formulation would have the advantage of ease of application, the powder simply being sprinkled into the wound. For the best results, such a powder should be both homogeneous and free flowing.

Unfortunately, most iodophors, especially the "non-ionic" variety (e.g. polydextrose iodine and povidone iodine) are amorphous, hygroscopic and non-free flowing powders. They can be transformed into free flowing powders by the addition of glidants, such as talc, but, although talc is widely used as a dusting powder and is an innocuous substance when applied to the intact skin, it may induce severe granulomatous reactions, when introduced into wounds or when employed during surgery, and so it cannot be considered as an ingredient in wound powders.

There is therefore a need for a wound powder containing an iodophor, but not talc, that is free flowing and homogeneous.

It is one object of the present invention to provide a process for the preparation of such a wound powder. Other objects and advantages of the present invention will become apparent from the following detailed description thereof.

According to the present invention there is provided a process for the preparation of a free flowing, iodophor containing wound powder comprising spraying a finely divided sugar in a fluidised bed granulator with a solution of the iodophor in a solvent comprising water and an alkyl alcohol having a boiling point, at 760mm Hg, below 100ºC.

The sugar employed in the present process may be any solid saccharide that can be finely divided enough to form a free flowing wound powder in the present process. Suitable sugars include sucrose, dextrose, maltose, fructose and lactose, with sucrose being particularly preferred.

The finely divided sugar may have any range of particle size that allows the formation of a finely divided wound powder by the present process. Preferably, however, all of the sugar will have particle sizes of 250 $\mu$m or less (60 mesh sieve), whilst at least 90% (by wt.) of the sugar will have particle sizes of 150 $\mu$m or less (100 mesh sieve).

The iodophor must form a free flowing powder in the present process. The most suitable iodophors are complexes of iodine with non-ionic, non-detergent organic polymers, especially polydextrose iodine or, which is particularly preferred, povidone iodine.

Povidone iodine is a well known iodophor that is a highly effective germicide, providing a broad spectrum of microbiocidal action against virtually all microbes. It may be prepared by any of a number of known routes, see, for example, European Published Applications No. EP-A-120301A and EP-A-6340A and British Patent No. GB-A-1580596.

Polydextrose is a non-nutritive polysaccharide, prepared by the condensation polymerisation of saccharides in the presence of polycarboxylic acid catalysts, under reduced pressure. Polydextrose is described in US Patents No. US-A-3766105 and US-A-3786794, and is available from Pfizer Inc., New York. Commercially available polydextrose polymer is a low molecular weight, water-soluble, randomly bonded polymer of glucose containing minor amounts of sorbitol end groups and citric acid residues attached to the polymer by mono- and di-ester bonds. The number average molecular weight of this commercially available material is 1,500, ranging from 160 to 20,000.

When polydextrose polymer is combined with elemental iodine, preferably in the presence of an alkali metal iodide, the resultant polydextrose iodine complex is formed. This complex is a tan-to-amber coloured product which melts between 90ºC and 130ºC to form a red liquid. Polydextrose iodine powder is highly soluble in water and at room temperature results in a reddish brown coloured aqueous solution.

The amount of iodine incorporated in the iodophors used in the present wound powder will be determined by, amongst other factors, the amount of iodophor present in the wound powder and the required antibacterial strength of the powder. Preferably, iodine will constitute between 1 and 20% (by wt.), especially between 2 and 15% (by wt.) of the iodophor dry weight.

2

The concentration of the iodophor in the present wound powder will depend on the antibacterial strength required. In addition, iodophor concentration will be determined by, amongst other factors, the iodophor employed, the propensity of the iodophor to cause irritation and the amount of iodine in the iodophor.

Thus, the wound powder preferably contains enough iodophor to afford a concentration of available (titratable) iodine within the powder of between 0.1 and 2% (by wt.), especially between 0.2 and 1.5% (by wt.).

Thus, a wound powder employing povidone iodine, with 10% (by wt.) available iodine, as the iodophor, would preferably contain between 1 and 20%, especially 2 and 15%, (by wt.) of povidone iodine.

Any alkyl alcohol having a boiling point, at 760mm Hg, below 100¤C, may be employed in the present process. Alkyl alcohols, such as methanol, ethanol and isopropanol, having a boiling point (at 760mm Hg) below 90¤C are preferred, with ethanol being particularly preferred.

Any ratio of alcohol to water in the present solvent that produces a free-flowing wound powder by the present process may be employed. The ratio is chosen so that the evaporation of the solvent is neither too slow nor too rapid. If the evaporation were too slow it would lead to sticky products, whereas, if it were too rapid, it would lead to non-homogeneous products. The present inventor has found that the most effective solvent for use in the present process contains
between 70% and 85% (v/v) alkyl alcohol and
between 30% and 15% (v/v) water, especially
between 78% and 80% (v/v) alkyl alcohol and
between 22% and 20% (v/v) water.

The iodophor solution may also contain selected pharmaceutical excipients that will facilitate the removal of the subsequent wound powder from a wound for cleansing or inspection purposes. These excipients are preferably free flowing powders and water soluble. Examples include polyethylene glycol and certain celluloses, e.g. carboxyalkylcelluloses and hydroxyalkylcelluloses.

The present process may be conducted at any temperature that leads to a free-flowing wound powder. In a preferred embodiment of the present invention, the process is conducted at a temperature between 30¤C and 50¤C, especially at about 40¤C.

It is an important feature of the present process that the pH of the subsequent iodophor/sugar combination may be adjusted to a value that is well tolerated in the treatment of wounds. Well tolerated iodophor/sugar powders will produce 50% (w/v) aqueous solutions that have a pH between 3.0 and 7.0, especially between 4.0 and 6.0.

This adjustment may be effected by the addition of a base to the iodophor solution prior to spraying. In a particularly preferred embodiment of the present process, a solution of povidone iodine in aqueous ethyl alcohol is neutralised with sodium hydroxide solution. In this embodiment, once the process is complete, a 50% (w/v) aqueous solution of the subsequent povidone iodine/sucrose powder has a pH between 4.5 and 5.0. It is therefore well tolerated when sprayed on open wounds.

Such an adjustment is not possible when the wound powder is prepared by conventional granulation methods or if an iodophor/sugar mixture is sprayed with dilute alcohol in a fluidised bed granulator. In these cases, a 50% (w/v) aqueous solution of a subsequent povidone iodine/sucrose powder has a pH value of 1.5. The powder would therefore not be well tolerated when sprayed on an open wound.

The production of a free flowing wound powder by the present process is surprising because

(a) When conventional granulation techniques are employed, that is mixing an iodophor (especially povidone iodine) and a sugar (especially sucrose) in a suitable granulator with the aid of an aqueous alcohol, subsequent drying and sieving produces a powder of insufficient homogeneity and fineness for wound treatment, and

(b) Furthermore, when a mixture of an iodophor (especially povidone iodine) and a sugar (especially sucrose) are sprayed with an aqueous alcohol in a fluidised bed granulator the product is once again non-homogeneous and too coarse.

Wound powders prepared by the present process may be dispersed freely onto skin or wounds to the skin. In order to facilitate the use of the present wound powders, however, they may be coated or admixed with other ingredients, such as free flowing powders, that are adapted to improve the therapeutic nature of the powder. Examples of such additional powders include

(i) Polyethylene glycol, a carboxyalkylcellulose or a hydroxyalkylcellulose. These facilitate the removal of a wound powder of the present type from a wound when it (the wound) has to be cleaned or inspected,

(ii) Zinc oxide. This material possesses mild astringent and antiseptic properties in its own right and therefore acts so as to aid the healing of skin wounds. Zinc oxide also improves the powder flow characteristics of the present wound powders.

3

Wound powders of the present invention are conveniently dispersed or sprinkled onto an area of skin to be treated from a bottle having a plurality of small holes at its outlet, such as the type of bottle that is generally used for the application of talcum powder. Preferably the bottle used is made of a flexible or resilient material so that the bottle may be squeezed to help expel the powder.

The present process and wound powders prepared by the present process will now be described by way of example only.

Preparation of Polydextrose Iodine Powder

Polydextrose (0.84 kg) was dissolved in warm water (10 litres). A solution of iodine (103gm) and ammonium iodide (57gm) in ethanol (3 litres) was added. After stirring for 1hr. at 20$\Box$C, the solvents were evaporated, under reduced pressure, and the residue was pulverised.

Example 1

Finely divided sucrose (4.5kg) was brought into a fluidised bed granulator at 40$\Box$C. Povidone iodine (0.5kg) was dissolved in a mixture of 96% ethanol (3.5 litres) and distilled water (0.75 litres). This solution was neutralised with 4N sodium hydroxide solution and then sprayed onto the sucrose powder at a rate of 50mlmin$^{-1}$ and at a spraying pressure of 4 bar (4 x 10$^5$ Pascals).

The granulate obtained was sieved through a 60 mesh sieve in order to remove any coarse particles. The yellow-brown, free-flowing, homogenous powder has a particle size distribution as follows:

```
60 mesh,               4% (by wt)   (250 μm)
80 mesh,               9%           (177 μm)
100 mesh,             43%           (150 μm)
120 mesh and finer,   44%           (125 μm)
```

Example 2

The process of Example 1 was repeated except that polydextrose iodine (0.4kg) replaced the povidone iodine.

Example 3

The process of Example 1 was repeated except that finely divided lactose (4.5kg) replaced sucrose as the sugar.

Example 4

The process of Example 1 was repeated except that finely divided dextrose (2.3kg) replaced sucrose as the sugar.

Example 5

Finely divided sucrose (4.8kg) was brought into a fluidised bed granulator at 40$\Box$C. Povidone iodine (0.6kg) was dissolved in 96% ethanol (3.5 litres) and a solution of polyethylene glycol 6000 (0.6kg) in distilled water (0.5 litres) was added. This solution was neutralised with 4N sodium hydroxide to pH 7.0 and then sprayed onto the sucrose powder at a rate of 50ml min$^{-1}$ and at a spraying pressure of 4 bar (4 x 10$^5$

Pascals).

## Claims

1. A process for the preparation of a free flowing, iodophor containing wound powder comprising spraying a finely divided sugar in a fluidised bed granulator with a solution of the iodophor in a solvent comprising water and an alkyl alcohol having a boiling point, at 760mm Hg, below 100°C.

2. A process according to claim 1 characterised in that the sugar comprises dextrose, maltose, fructose, lactose or, preferably, sucrose.

3. A process according to either claim 1 or claim 2 characterised in that all of the sugar has a particle size of 250 $\mu$m (microns) or less and in that 90% (by wt) of the sugar has a particle size of 150 $\mu$m (microns) or less.

4. A process according to any one of claims 1 to 3 characterised in that the iodophor comprises polydextrose iodine or, preferably, povidone iodine.

5. A process according to any one of claims 1 to 4 characterised in that the amount of iodophor in the solvent is sufficient to give a wound powder containing between 0.1 and 2.0% (by wt), especially between 0.2 and 1.5% (by wt), available iodine.

6. A process according to any one of claims 1 to 5 characterised in that the alkyl alcohol comprises methanol, isopropanol or, preferably, ethanol.

7. A process according to any one of claims 1 to 6 characterised in that the solvent comprises between 70 and 85% (v/v) alkyl alcohol and between 30 and 15% (v/v) water, preferably between 78 and 80% (v/v) alkyl alcohol and between 22 and 20% (v/v) water.

8. A process according to any one of claims 1 to 7 characterised in that, before being sprayed onto the finely divided sugar, the pH of the iodophor solution is adjusted such that a 50% (w/v) aqueous solution of the subsequent wound powder has a pH between 3.0 and 7.0, preferably between 4.0 and 6.0.

9. A process according to any one of claims 1 to 8 characterised in that the wound powder obtained is subsequently coated or admixed with a free flowing powder selected from polyethylene glycol, a carboxymethylcellulose, a hydroxyalkylcellulose or zinc oxide.

## Revendications

1. Procédé de préparation d'une poudre cicatrisante s'écoulant librement, contenant un iodophore, qui comprend la pulvérisation, sur un sucre finement divisé dans un granulateur à lit fluidisé, d'une solution de l'iodophore dans un solvant comprenant de l'eau et un alcool alkylique ayant un point d'ébullition, sous 760 mm de Hg, inférieur à 100° C.

2. Procédé selon la revendication 1, caractérisé en ce que le sucre englobe le dextrose, le maltose, le fructose, le lactose ou, de préférence, le saccharose.

3. Procédé selon l'une quelconque des revendications 1 ou 2, caractérisé en ce que la totalité du sucre a une granulométrie inférieure ou égale à 250 $\mu$m (microns) et en ce que 90% (en poids) du sucre a une granulométrie inférieure ou égale à 150 $\mu$m (microns).

4. Procédé selon l'une quelconque des revendications 1 à 3, caractérisé en ce que l'iodophore englobe le polydextrose-iode ou, de préférence, le povidone-iode.

5. Procédé selon l'une quelconque des revendications 1 à 4, caractérisé en ce que la quantité d'iodophore dans le solvant est suffisante pour donner une poudre cicatrisante contenant entre 0,1 et 2,0% (en poids), en particulier entre 0,2 et 1,5% (en poids), d'iode disponible.

6. Procédé selon l'une quelconque des revendications 1 à 5, caractérisé en ce que l'alcool alkylique englobe le méthanol, l'isopropanol ou, de préférence, l'éthanol.

7. Procédé selon l'une quelconque des revendications 1 à 6, caractérisé en ce que le solvant comprend entre 70 et 85% (en volume) d'alcool alkylique et entre 30 et 15% (en volume) d'eau, de préférence entre 78 et 80% (en volume) d'alcool éthylique et entre 22 et 20% (en volume) d'eau.

8. Procédé selon l'une quelconque des revendications 1 à 7, caractérisé en ce que, avant de la pulvériser sur le sucre finement divisé, on ajuste le pH de la solution d'iodophore de telle manière qu'une solution aqueuse à 50% (en poids/volume) de la poudre cicatrisante qui en résulte ait un pH compris entre 3,0 et 7,0, de préférence entre 4,0 et 6,0.

9. Procédé selon l'une quelconque des revendications 1 à 8, caractérisé en ce que la poudre cicatrisante obtenue est ensuite appliquée ou mélangée avec une poudre s'écoulant librement choisie parmi un polyéthylèneglycol, une carboxyméthylcellulose, une hydroxyalkylcellulose ou l'oxyde de zinc.

**Ansprüche**

1. Verfahren zum Herstellen eines freifließenden, jodophorhaltigen Wundpulvers, bei welchem ein feinteiliger Zucker zusammen mit einer Lösung des Jodophors in einem Wasser und einen Alkylalkohol mit einem unter 100 °C bei 760 mmHg liegenden Siedepunkt besitzenden-Lösungsmittel in einen Fließbettgranulator gesprüht wird.

2. Verfahren nach Anspruch 1, dadurch gekennzeichnet, daß der Zucker Dextrose, Maltose, Fructose, Lactose oder, vorzugsweise, Saccharose umfaßt.

3. Verfahren nach Anspruch 1 oder Anspruch 2, dadurch gekennzeichnet, daß der gesamte Zucker eine Teilchengröße von 250 μm (Micron) oder weniger besitzt und daß 90 Gew.-% des Zuckers eine Teilchengröße von 150 μm (Micron) oder weniger besitzt.

4. Verfahren nach irgendeinem der Ansprüche 1 bis 3, dadurch gekennzeichnet, daß der Jodophor Polydextrose-Jod oder, vorzugsweise, Povidone-Jod umfaßt.

5. Verfahren nach irgendeinem der Ansprüche 1 bis 4, dadurch gekennzeichnet, daß die Menge an Jodophor im Lösungsmittel ausreicht ein Wundpulver zu ergeben, welches zwischen 0,1 und 2,0 Gew.-%, insbesondere zwischen 0,2 und 1,5 Gew.-%, verfügbares Jod enthält.

6. Verfahren nach irgendeinem der Ansprüche 1 bis 5, dadurch gekennzeichnet, daß der Alkylalkohol Methanol, Isopropanol oder, vorzugsweise, Äthanol umfaßt.

7. Verfahren nach irgendeinem der Ansprüche 1 bis 6, dadurch gekennzeichnet, daß das Lösungsmittel zwischen 70 und 85 Vol.-% Alkylalkohol und zwischen 30 und 15 Vol.-% Wasser, vorzugsweise zwischen 78 und 80 Vol.-% Alkylalkohol und zwischen 22 und 20 Vol.-% Wasser, enthält.

8. Verfahren nach irgendeinem der Ansprüche 1 bis 7, dadurch gekennzeichnet, daß der pH-Wert der Jodophorlösung vor dem Aufsprühen auf den fein verteilten Zucker so eingestellt wird, daß eine 50%ige (w/v) wässerige Lösung des erhaltenen Wundpulvers einen pH-Wert zwischen 3,0 und 7,0, vorzugsweise zwischen 4,0 und 6,0, besitzt.

9. Verfahren nach irgendeinem der Ansprüche 1 bis 8, dadurch gekennzeichnet, daß das erhaltene Wundpulver anschließend mit einem aus Polyäthylenglykol, einer Carboxymethylcellulose, einer Hydroxyalkylcellulose oder Zinkoxid ausgewählten freifließenden Pulver beschichtet oder vermischt wird.